# EUROPÄISCHE PATENTANMELDUNG

(11) **EP 0 785 200 A2**
(43) Veröffentlichungstag der Anmeldung: **23.07.1997**
(21) Anmeldenummer: 97100025.2
(22) Anmeldetag: 03.01.1997
(51) Int. Cl.: C07D 495/04, A61K 31/44, A61K 31/49

(54) **Pyrido-annellierte Thienyl- und Furanyl-Oxazolidinone**

(30) Priorität: 16.01.1996 DE 19601264
(71) Anmelder: BAYER AG, 51368 Leverkusen (DE)
(72) Erfinder: Riedl, Bernd, Dr., 42329 Wuppertal (DE); Häbich, Dieter, Dr., 42115 Wuppertal (DE); Stolle, Andreas, Dr., 42115 Wuppertal (DE); Ruppelt, Martin, Dr., 42115 Wuppertal (DE); Bartel, Stephen, Dr, 51465 Bergisch Gladbach (DE); Guarnieri, Walter, Dr., 53909 Zülpich (DE); Endermann, Rainer, Dr., 42113 Wuppertal (DE); Kroll, Hein-Peter, Dr., 42115 Wuppertal (DE)

(57) **Zusammenfassung**

Die vorliegende Erfindung betrifft neue Pyrido-annellierte Thienyl- und Furanyl-Oxazolidinone, Verfahren zu ihrer Herstellung und ihre Verwendung als Arzneimittel, insbesondere als antibakterielle Arzneimittel.

## Beschreibung

Die vorliegende Erfindung betrifft neue Pyrido-annellierte Thienyl- und Furanyl-Oxazolidinone, Verfahren zu ihrer Herstellung und ihre Verwendung als Arzneimittel, insbesondere als antibakterielle Arzneimittel.

N-Aryloxazolidinone mit antibakterieller Wirkung sind beispielsweise aus den Publikationen EP 311 090 und US 4 705 799 bekannt. Außerdem sind 3-(Stickstoff-substituierte)phenyl-5-beta-amidomethyloxazolidin-2-one aus der EP 609 905 A1 bekannt.

Ferner sind unter anderem in der WO 93 08 179 A Oxazolidinonderivate mit einer Monoaminoxidase inhibitorischen Wirkung und in der EP 645 376 mit Wirkung als Adhäsionsrezeptor-Antagonisten publiziert.

Die vorliegende Erfindung betrifft Pyrido-annellierte Thienyl- und Furanyl-Oxazolidinone der allgemeinen Formel (I) 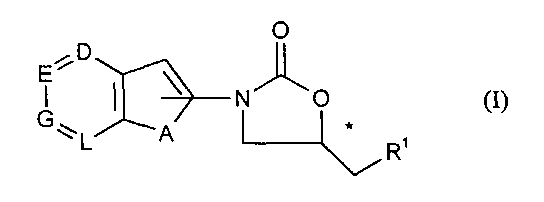 in welcher
- A: für ein Sauerstoff- oder Schwefelatom oder für die SO₂-Gruppe steht,
und
- D, E, G und L: gleich oder verschieden sind und mindestens einer dieser Substituenten für ein Stickstoffatom steht und die übrigen für einen Rest der Formel -CR² stehen,
worin
- R²: Wasserstoff, Cyano, Nitro, Carboxyl, geradkettiges oder verzweigtes Alkyl, Acyl oder Alkoxy mit jeweils bis zu 7 Kohlenstoffatomen, Halogen oder eine Gruppe der Formel -NR³R⁴, -CO-NR⁵R⁶, -NR⁷-CO-R⁸ oder -S(O)ₐR⁹ bedeutet,
worin
- R³, R⁴, R⁵, R⁶, R⁷ und R⁸: gleich oder verschieden sind und Wasserstoff, Phenyl oder geradkettiges oder verzweigtes Alkyl mit bis zu 6 Kohlenstoffatomen bedeuten,
- a: eine Zahl 0, 1 oder 2 bedeutet,
- R⁹: Phenyl oder geradkettiges oder verzweigtes Alkyl mit bis zu 4 Kohlenstoffatomen bedeutet,
- R¹: für Azido, Hydroxy oder für eine Gruppe der Formel -OR¹⁰, O-SO₂R¹¹ oder -NR¹²R¹³ steht,
worin
- R¹⁰: geradkettiges oder verzweigtes Acyl mit bis zu 8 Kohlenstoffatomen oder eine Hydroxyschutzgruppe bedeutet,
- R¹¹: geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,
- R¹² und R¹³: gleich oder verschieden sind und Cycloalkyl mit 3 bis 6 Kohlenstoffatomen, Wasserstoff, Phenyl oder geradkettiges oder verzweigtes Alkyl oder Alkoxy mit jeweils bis zu 8 Kohlenstoffatomen oder eine Aminoschutzgruppe bedeuten,
oder
- R¹² oder R¹³: eine Gruppe der Formel -CO-R¹⁴, -CS-R^{14'}, P(O)(OR¹⁵)(OR¹⁶) oder -SO₂-R¹⁷ bedeutet,
worin
- R¹⁴ und R^{14'}: gleich oder verschieden sind und Cycloalkyl mit 3 bis 6 Kohlenstoffatomen, Trifluormethyl, geradkettiges oder verzweigtes Alkoxy mit bis zu 8 Kohlenstoffatomen, Phenyl, Benzyloxy oder Wasserstoff bedeuten, oder
- R¹⁴ und R^{14'}: geradkettiges oder verzweigtes Alkyl mit bis zu 8 Kohlenstoffatomen bedeuten, das gegebenenfalls durch Cyano, Halogen oder Trifluormethyl substituiert ist,
oder
geradkettiges oder verzweigtes Thioalkyl oder Acyl mit jeweils bis zu 6 Kohlenstoffatomen bedeuten,
oder
eine Gruppe der Formel -NR¹⁸R¹⁹ bedeuten,
worin
- R¹⁸ und R¹⁹: gleich oder verschieden sind und Wasserstoff, Phenyl oder geradkettiges oder verzweigtes Alkyl mit bis zu 6 Kohlenstoffatomen bedeuten,
oder
einen 5-gliedrigen aromatischen Heterocyclus mit bis zu 3 Heteroatomen aus der Reihe S, N und/oder O bedeuten,
- R¹⁵ und R¹⁶: gleich oder verschieden sind und Wasserstoff oder geradkettiges oder verzweigtes Alkyl mit bis zu 4 Kohlenstoffatomen bedeuten,
- R¹⁷: geradkettiges oder verzweigtes Alkyl mit bis zu 4 Kohlenstoffatomen oder Phenyl bedeutet
und deren Salze.

Physiologisch unbedenkliche Salze der neuen Pyrido-annellierten Thienyl- und Furanyl-Oxazolidinone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können weiterhin Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methyl-piperidin.

Als Salze können außerdem Reaktionsprodukte mit C₁-C₄-Alkylhalogeniden, insbesondere C₁-C₄-Alkyljodiden fungieren.

Hydroxyschutzgruppe im Rahmen der oben angegebenen Definition steht im allgemeinen für eine Schutzgruppe aus der Reihe: Trimethylsilyl, Triisopropylsilyl, tert.Butyl-dimethylsilyl, Benzyl, Benzyloxycarbonyl, 2-Nitrobenzyl, 4-Nitrobenzyl, tert. Butyloxycarbonyl, Allyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl)ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, tert. Butyldimethylsilyl und Tetrahydropyranyl.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 2,4-Dimethoxybenzyloxycarbonyl, 4-Methoxybenzyloxycarbonyl, Methoxycarbonyl, Ethoxycarbonyl, tert.Butoxycarbonyl, Allyloxycarbonyl, Phthaloyl, 2,2,2-Trichlorethoxycarbonyl, Fluorenyl-9-methoxycarbonyl, Formyl, Acetyl, 2-Chloracetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl, 4-Methoxyphenyl oder Triphenylmethyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher
- A: für ein Sauerstoff- oder Schwefelatom oder für die -SO₂-Gruppe steht,
und
- D, E, G und L: gleich oder verschieden sind und mindestens einer dieser Substituenten für ein Stickstoffatom steht und die übrigen für einen Rest der Formel -CR² stehen,
worin
- R²: Wasserstoff, geradkettiges oder verzweigtes Alkyl mit bis zu 6 Kohlenstoffatomen, Fluor, Chlor oder Brom bedeutet,
- R¹: für Azido, Hydroxy oder für eine Gruppe der Formel -OR¹⁰, O-SO₂R¹¹ oder -NR¹²R¹³ steht,
worin
- R¹⁰: geradkettiges oder verzweigtes Acyl mit bis zu 6 Kohlenstoffatomen oder Benzyl bedeutet,
- R¹¹: geradkettiges oder verzweigtes Alkyl mit bis zu 3 Kohlenstoffatomen, Phenyl oder Tolyl bedeutet,
- R¹² und R¹: ³ gleich oder verschieden sind und Cyclopropyl, Cyclopentyl, Cyclohexyl, Wasserstoff, Phenyl oder geradkettiges oder verzweigtes Alkyl oder Alkoxy mit jeweils bis zu 6 Kohlenstoffatomen, tert.Butoxycarbonyl oder Benzyloxycarbonyl bedeuten,
oder
- R¹² oder R¹³: eine Gruppe der Formel -CO-R¹⁴, -CS-R^{14'}, P(O)(OR¹⁵)(OR¹⁶) oder -SO₂-R¹⁷ bedeutet,
worin
- R¹⁴ und R^{14'}: gleich oder verschieden sind und Cyclopropyl, Cyclobutyl, Cyclopentyl, Cyclohexyl, Trifluormethyl oder geradkettiges oder verzweigtes Alkoxy mit bis zu 6 Kohlenstoffatomen, Phenyl, Benzyloxy oder Wasserstoff bedeuten, oder
- R¹⁴ und R^{14'}: geradkettiges oder verzweigtes Alkyl mit bis zu 6 Kohlenstoffatomen bedeuten, das gegebenenfalls durch Cyano, Fluor, Chlor, Brom oder Trifluormethyl substituiert ist, oder
geradkettiges oder verzweigtes Thioalkyl oder Acyl mit jeweils bis zu 5 Kohlenstoffatomen bedeuten, oder
eine Gruppe der Formel -NR¹⁸R¹⁹ bedeuten,
worin
- R¹⁸ und R¹⁹: gleich oder verschieden sind und Wasserstoff, Phenyl oder geradkettiges oder verzweigtes Alkyl mit bis zu 4 Kohlenstoffatomen bedeuten,
oder
Isoxazolyl, Furyl, Thienyl, Pyrryl, Oxazolyl oder Imidazolyl bedeuten,
- R¹⁵ und R¹⁶: gleich oder verschieden sind und Wasserstoff oder geradkettiges oder verzweigtes Alkyl mit bis zu 3 Kohlenstoffatomen bedeuten,
- R¹⁷: geradkettiges oder verzweigtes Alkyl mit bis zu 3 Kohlenstoffatomen oder Phenyl bedeutet
und deren Salze.

Besonders bevorzugt sind Verbindungen der allgemeinen Formel (I),
in welcher
- A: für ein Sauerstoff- oder Schwefelatom oder für die -SO₂-Gruppe steht,
und
- D, E, G und L: gleich oder verschieden sind und mindestens einer dieser Substituenten für ein Stickstoffatom steht und die übrigen für einen Rest der Formel -CR² stehen,
worin
- R²: Wasserstoff, geradkettiges oder verzweigtes Alkyl mit bis zu 3 Kohlenstoffatomen oder Fluor bedeutet,
- R¹: für Azido, Hydroxy oder für eine Gruppe der Formel -OR¹⁰, O-SO₂R¹¹ oder -NR¹²R¹³ steht,
worin
- R¹⁰: geradkettiges oder verzweigtes Acyl mit bis zu 5 Kohlenstoffatomen oder Benzyl bedeutet,
- R¹¹: Methyl, Ethyl, Phenyl oder Toluolyl bedeutet,
- R¹² und R¹³: gleich oder verschieden sind und Cyclopropyl, Cyclopentyl, Cyclohexyl, Wasserstoff, Phenyl oder geradkettiges oder verzweigtes Alkyl oder Alkoxy mit jeweils bis zu 5 Kohlenstoffatomen, tert.Butoxycarbonyl oder Benzyloxycarbonyl bedeuten,
oder
- R¹² oder R¹³: eine Gruppe der Formel -CO-R¹⁴, -CS-R^{14'}, P(O)(OR¹⁵)(OR¹⁶) oder -SO₂R¹⁷ bedeutet,
worin
- R¹⁴ und R^{14'}: gleich oder verschieden sind und Cyclopropyl, Cyclobutyl, Cyclopentyl, Cyclohexyl, Trifluorethyl oder geradkettiges oder verzweigtes Alkoxy mit bis zu 5 Kohlenstoffatomen, Phenyl, Benzyloxy oder Wasserstoff bedeuten,
oder
- R¹⁴ und R^{14'}: geradkettiges oder verzweigtes Alkyl mit bis zu 5 Kohlenstoffatomen bedeuten, das gegebenenfalls durch Cyano, Fluor, Chlor, Brom oder Trifluormethyl substituiert ist, oder
geradkettiges oder verzweigtes Thioalkyl oder Acyl mit jeweils bis zu 4 Kohlenstoffatomen bedeuten, oder
eine Gruppe der Formel -NR¹⁸R¹⁹ bedeuten,
worin
- R¹⁸ und R¹⁹: gleich oder verschieden sind und Wasserstoff, Phenyl oder geradkettiges oder verzweigtes Alkyl mit bis zu 3 Kohlenstoffatomen bedeuten, oder
Isoxazolyl, Furyl, Oxazolyl oder Imidazolyl bedeuten,
- R¹⁵ und R¹⁶: gleich oder verschieden sind und Wasserstoff, Methyl oder Ethyl bedeuten,
- R¹⁷: Methyl oder Phenyl bedeutet
und deren Salze.

Ganz besonders bevorzugt sind die erfindungsgemäßen Verbindungen der allgemeinen Formel (I),
in welcher
der Oxazolidinonrest in der Position 2 am 5-Ring-Heterocyclus angebunden ist.

Außerdem wurden Verfahren zur Herstellung der erfindungsgemäßen Verbindungen der allgemeinen Formel (I) gefunden, dadurch gekennzeichnet, daß man
[A] Verbindungen der allgemeinen Formel (II) oder (III) 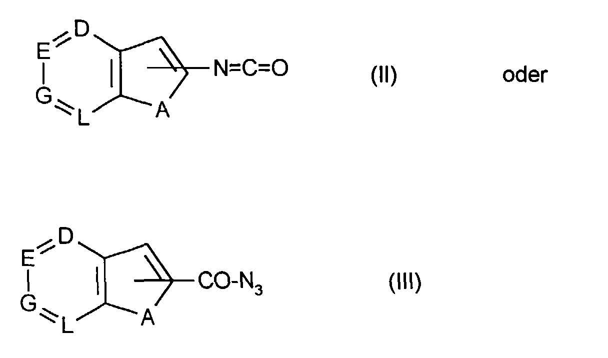 in welchen
   - A, D, E, G und L: die oben angegebene Bedeutungen haben,
   mit Lithiumbromid/(C₄H₉)₃P(O) und Epoxiden der allgemeinen Formel (IV) 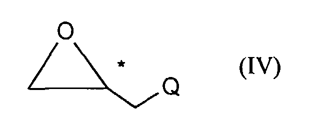 in welcher
   - Q: für C₁-C₆-Acyloxy steht,
   in inerten Lösemitteln, gegebenenfalls in Anwesenheit einer Base
   umsetzt,
   und im Fall R¹ = OH durch eine typische Esterverseifung oder durch eine typische Umesterung die Hydroxyfunktion freisetzt,
   oder
[B] Verbindungen der allgemeinen Formel (V) 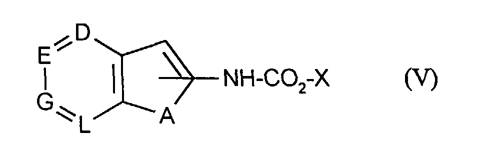 in welcher
   - A, D, E, G und L: die oben angegebene Bedeutung haben
   und
   - X: für eine typische Schutzgruppe, vorzugsweise Benzyl steht,
   in inerten Lösemitteln und in Anwesenheit einer Base, beispielsweise Lithiumalkylen oder Lithium-N-alkyl- oder Lithium-N-silylalkylamiden, vorzugsweise N-Butyllithium, mit Epoxiden der allgemeinen Formel (IV) umsetzt,
   oder
[C] im Fall R¹ = OH, zunächst Verbindungen der allgemeinen Formel (III) durch Abspaltung von Stickstoff in Alkoholen in die Verbindungen der allgemeinen Formel (Va) 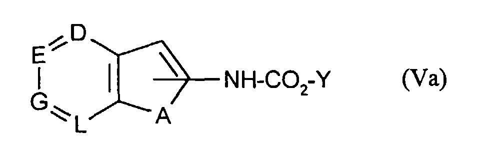 in welcher
   - A, D, E, G und L: die oben angegebene Bedeutung haben
   und
   - Y: für geradkettiges oder verzweigtes C₂-C₆-Alkyl, vorzugsweise n-Butyl steht,
   überführt,
   und in einem zweiten Schritt wie unter [A] beschrieben in inerten Lösemitteln und in Anwesenheit einer Base, vorzugsweise Lithium-N-alkyl- oder N-Silylalkylamiden oder n-Butyllithium mit Epoxiden der allgemeinen Formel (IV) umsetzt,
   oder
[D] Verbindungen der allgemeinen Formel (VI) 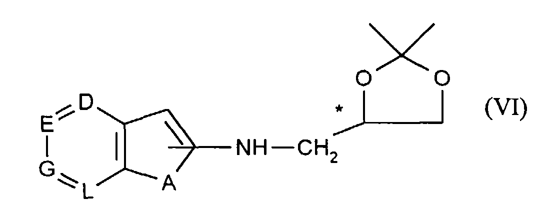 in welcher
   - A, D, E, G und L: die oben angegebene Bedeutung haben,
   entweder direkt mit Säuren und Kohlensäurediethylester
   umsetzt,
   oder zunächst durch Umsetzung der Verbindungen der allgemeinen Formel (VI) mit Säuren die Verbindungen der allgemeinen Formel (VII) 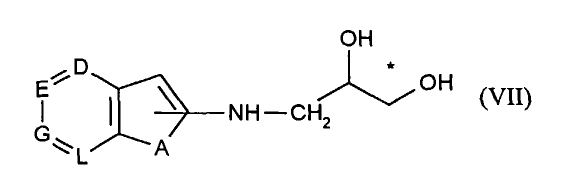 in welcher
   - A, D, E, G und L: die oben angegebene Bedeutung haben,
   herstellt,
   und anschließend in Anwesenheit eines Hilfsmittels in inerten Lösemitteln cyclisiert,
   oder
[E] zunächst Verbindungen der allgemeinen Formel (Ia) 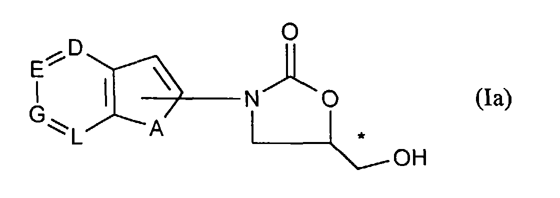 in welcher
   - A, D, E, G und L: die oben angegebene Bedeutung haben,
   durch Umsetzung mit (C₁-C₄)-Alkyl- oder Phenylsulfonsäurechloriden, die gegebenenfalls entsprechend substituiert sind, in inerten Lösemitteln und in Anwesenheit einer Base in die entsprechenden Verbindungen der allgemeinen Formel (Ib) 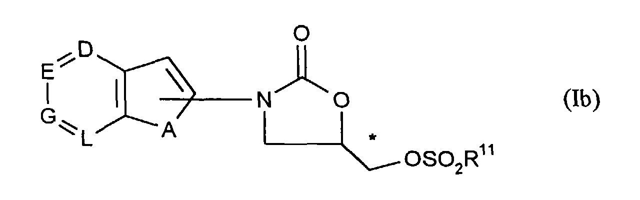 in welcher
   - A, D, E, G, L und R¹¹: die oben angegebene Bedeutung haben,
   überführt,
   anschließend mit Natriumazid in inerten Lösemitteln die Azide der allgemeinen Formel (Ic) 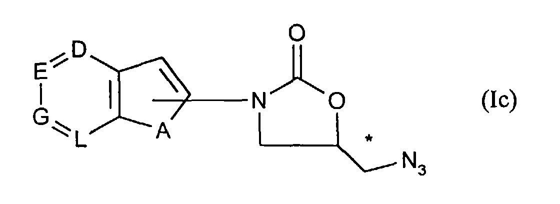 in welcher
   - A, D, E, G und L: die oben angegebene Bedeutung haben,
   herstellt,
   in einem weiteren Schritt durch Umsetzung mit (C₁-C₄-O)₃-P oder PPh₃, vorzugsweise (CH₃O)₃P in inerten Lösemitteln und mit Säuren in die Amine der allgemeinen Formel (Id) 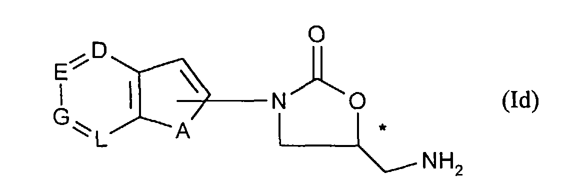 in welcher
   - A, D, E, G und L: die oben angegebene Bedeutung haben,
   überführt,
   und durch Umsetzung mit Acetanhydrid oder anderen Acylierungsmitteln der allgemeinen Formel (VIII)

   R²⁰-CO-R¹⁴ (VIII)

   in welcher
   - R¹⁴: die oben angegebene Bedeutung hat
   und
   - R²⁰: für Halogen, vorzugsweise für Chlor oder für den Rest -OCOR¹⁴ steht,
   in inerten Lösemitteln die Verbindungen der allgemeinen Formel (Ie) 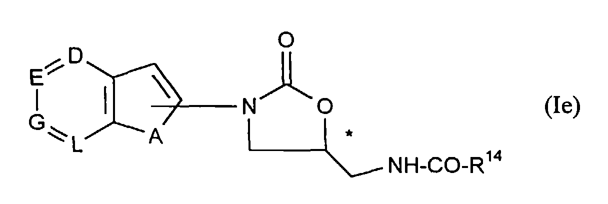 in welcher
   - A, D, E, G, L und R¹⁴: die oben angegebene Bedeutung haben,
   herstellt,
   und im Fall R¹ = NR¹²-CS-R^{14'} Verbindungen der allgemeinen Formel (Id) mit Ethyldithiocarboxylaten und Triethylamin und im Fall R¹ = NR¹²-CS-NR¹⁸R¹⁹ mit Thioisocyanaten umsetzt,
   und im Fall der S-Oxide eine Oxidation nach üblicher Methode durchführt,
   und gegebenenfalls weitere Substituenten oder bereits vorhandene funktionelle Gruppen nach üblichen Methoden, wie beispielsweise Alkylierung, Redoxreaktionen, Substitutionsreaktionen und/oder Verseifungen oder Ein- und Abbau von Schutzgruppen, einführt bzw. derivatisiert.

Die erfindungsgemäßen Verfahren können durch folgende Formelschemata beispielhaft erläutert werden:

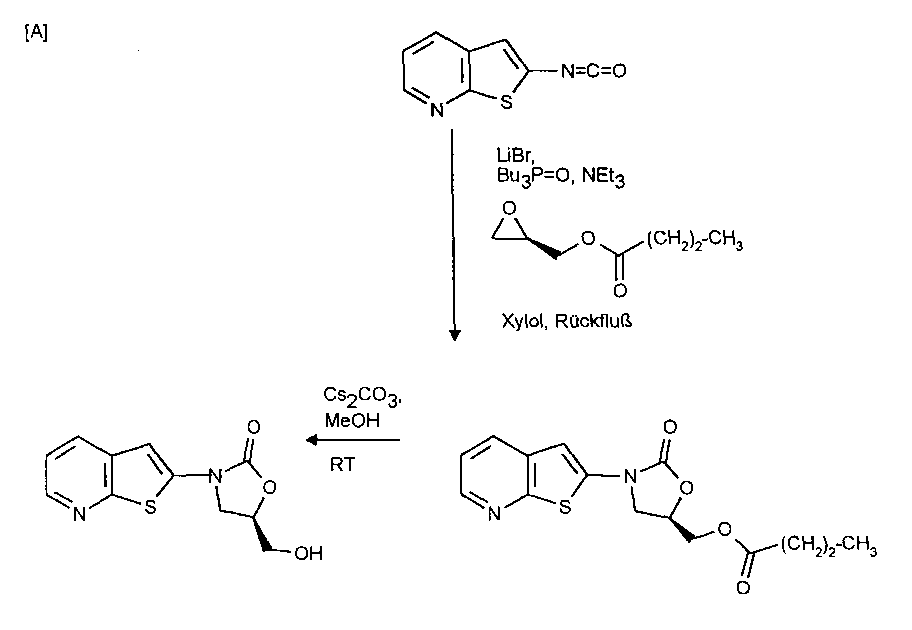

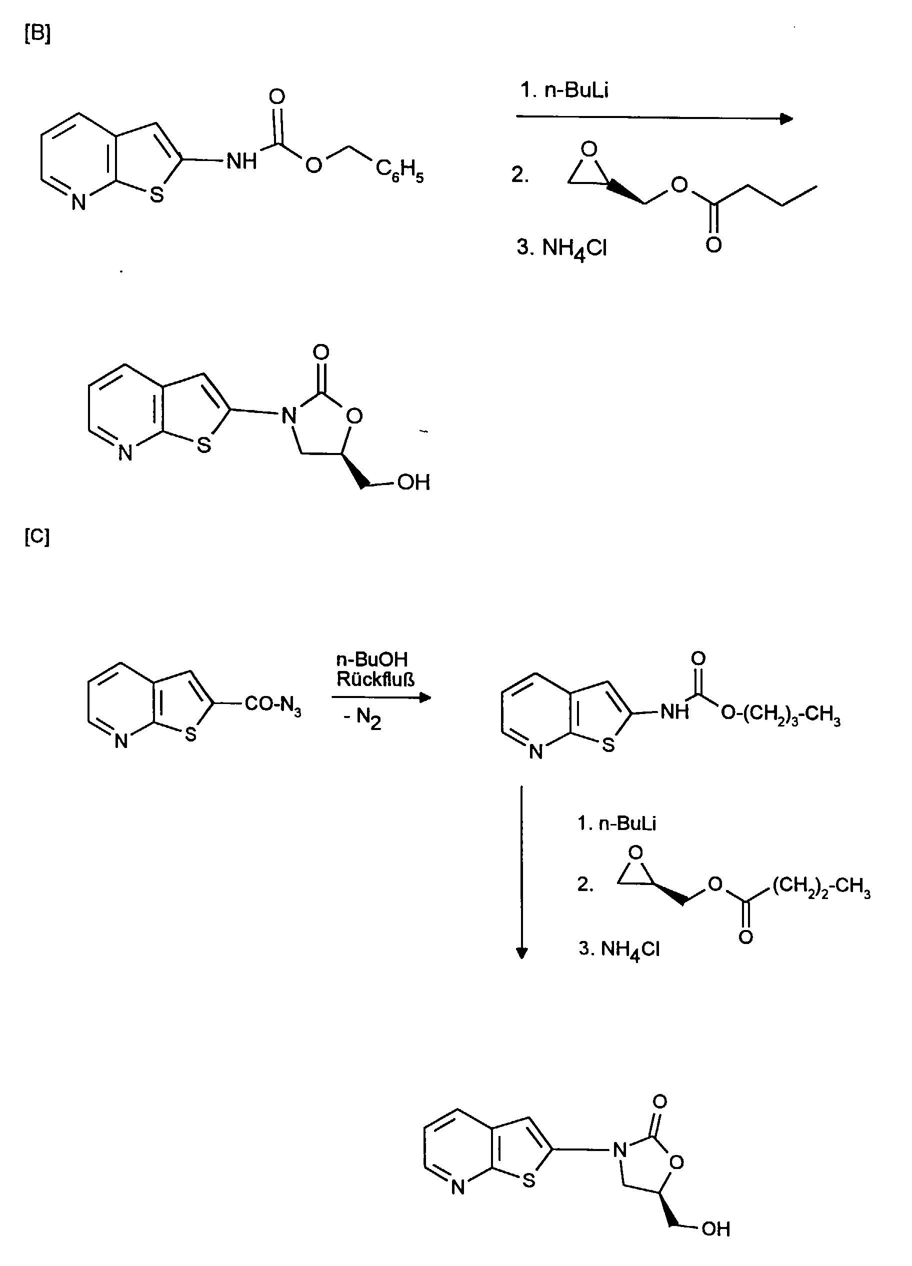

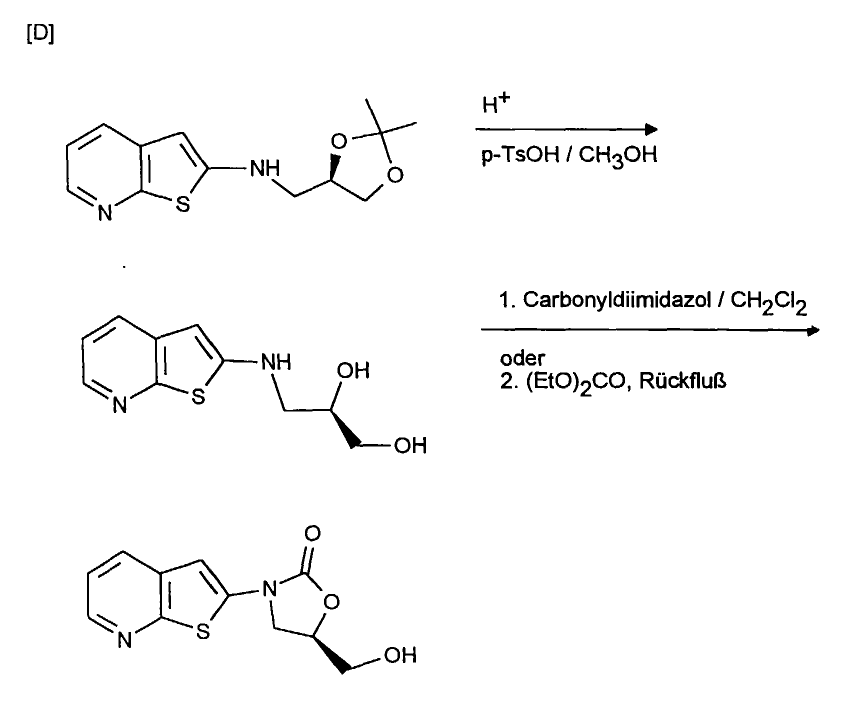

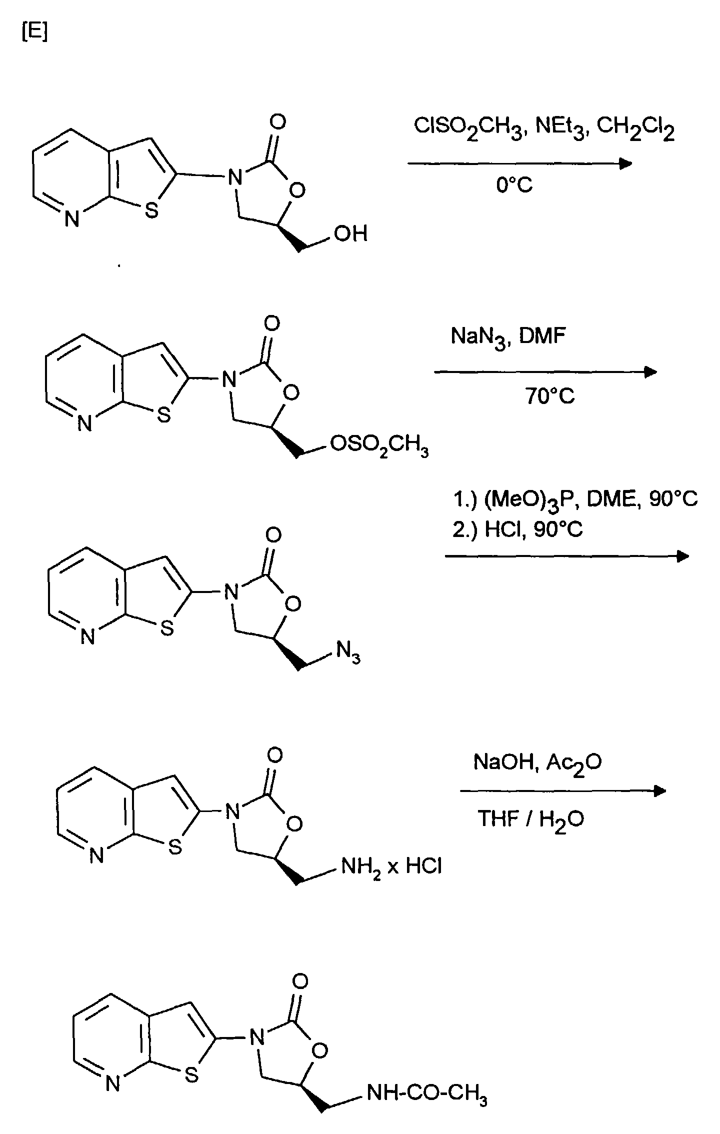

Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylalkylamide, wie beispielsweise Lithium-N-(bis)triphenylsilylamid oder Lithiumalkyle wie n-Butyllithium.

Die Base wird in einer Menge von 1 mol bis 10 mol, bevorzugt von 1 mol bis 3 mol bezogen auf 1mol der Verbindungen der allgemeinen Formeln (II), (III), (IV) und (Va) eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Das Verfahren [A] erfolgt bevorzugt in Xylol oder Dichlorbenzol, gegebenenfalls in Gegenwart von Triethylamin, unter Rückfluß.

Die basenkatalysierte Umesterung wird mit einem der oben aufgeführten Alkohole, vorzugsweise Methanol, in einem Temperaturbereich von -10°C bis +40°C, vorzugsweise bei Raumtemperatur durchgeführt.

Als Basen eignen sich im allgemeinen Natriumhydrogencarbonat, Natriummethanolat, Hydrazinhydrat, Kaliumcarbonat oder Caesiumcarbonat. Bevorzugt ist Caesiumcarbonat.

Das Verfahren [B] erfolgt in einem der oben aufgeführten Ether mit Lithiumalkylverbindungen oder Lithium-N-silylamiden, wie beispielsweise n-Butyllithium, Lithiumdiisopropylamid oder Lithium-bistrimethylsilylamid, vorzugsweise in Tetrahydrofuran und Lithium-bis-trimethylsilylamid oder n-Butyllithium, in einem Temperaturbereich von -100°C bis +20°C, vorzugsweise von -75°C bis -40°C.

Für das Verfahren [C] eignen sich für den 1. Schritt vorzugsweise die oben aufgeführten Alkohole, im Falle der anschließenden Cyclisierung Tetrahydrofuran.

Als Basen für die Cyclisierung eignen sich vorzugsweise die oben aufgeführten Lithium-N-silylalkylverbindungen oder n-Butyllithium. Besonders bevorzugt ist n-Butyllithium.

Der erste Reaktionsschritt wird bei der Siedetemperatur des entsprechenden Alkohols, die Cyclisierung in einem Temperaturbereich von -70°C bis Raumtemperatur durchgeführt.

Die Cyclisierung [D] wird in Anwesenheit eines Hilfsmittels und/oder Anwesenheit einer Säure durchgeführt.

Als Säuren eignen sich im allgemeinen anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure. Besonders bevorzugt ist Salzsäure.

Die Säure wird in einer Menge von 1 mol bis 10 mol, bevorzugt von 1 mol bis 2 mol, bezogen auf 1 mol der Verbindungen der allgemeinen Formel (VI) eingesetzt.

Als Hilfsmittel eignen sich die üblichen Reagenzien wie Phosgen, Carbonyldiimidazol oder Kohlensäurediethylester oder Chlorameisensäuretrichlormethylester. Bevorzugt sind Carbonyldiimidazol, Kohlensäurediethylester und Chlorameisensäuretrichlormethylester.

Als Lösemittel eignen sich die oben aufgeführten Halogenkohlenwasserstoffe. Bevorzugt ist Methylenchlorid.

Die Cyclisierungen erfolgen im allgemeinen in einem Temperaturbereich von -20°C bis 100°C, vorzugsweise bei -20°C bis Raumtemperatur.

Die Acylierung [E] erfolgt im allgemeinen in einem der oben aufgeführten Ether oder Halogenkohlenwasserstoffen, vorzugsweise Tetrahydrofuran oder Methylenchlorid, in einem Temperaturbereich von -30°C bis 50°C, bevorzugt von -10°C bis Raumtemperatur.

Die Reduktionen erfolgen im allgemeinen mit Hydriden in inerten Lösemitteln oder mit Boranen, Diboranen oder ihren Komplexverbindungen.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Bevorzugt werden die Reduktionen mit Hydriden, wie komplexen Borhydriden oder Aluminiumhydriden sowie Boranen durchgeführt. Besonders bevorzugt werden hierbei Natriumborhydrid, Lithiumborhydrid, Natriumcyanoborhydrid, Lithiumaluminiumhydrid, Natrium-bis-(2-methoxyethoxy)aluminiumhydrid oder Boran-Tetrahydrofuran eingesetzt.

Die Reduktion der Azide [E] erfolgt mit (CH₃O)₃P und Salzsäure.

Die Reduktion erfolgt im allgemeinen in einem Temperaturbereich von -50°C bis zum jeweiligen Siedepunkt des Lösemittels, bevorzugt von -20°C bis +90°C.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Die Abspaltung der Hydroxyschutzgruppen erfolgt im allgemeinen nach üblicher Methode, beispielsweise durch hydrogenolytische Spaltung der Benzylether in den oben aufgeführten inerten Lösemitteln in Anwesenheit eines Katalysators mit Wasserstoff-Gas.

Die Abspaltung der Aminoschutzgruppe erfolgt im allgemeinen ebenfalls nach üblichen Methoden, und zwar wird vorzugsweise Boc mit Salzsäure in Dioxan, Fmoc mit Piperidin und Z mit HBr/HOAc oder durch Hydrogenolyse abgespalten.

Bevorzugt werden Redoxreaktionen, reduktive Aminierung, Umesterung und die Halogenisierung von Methylgruppen mit N-Bromsuccinimid (NBS) oder N-Chlorsuccinimid (NCS) aufgeführt, die im folgenden beispielhaft erläutert werden.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Acetonitril, Aceton oder Nitromethan. Ebenso ist es möglich, Gemische der genannten Lösemittel zu verwenden. Bevorzugt sind Dichlormethan, Dimethylsulfoxid und Dimethylformamid.

Die Alkylierung wird in den oben aufgeführten Lösemitteln bei Temperaturen von 0°C bis +150°C, vorzugsweise bei Raumtemperatur bis +100°C, bei Normaldruck durchgeführt.

Die Amidierung und die Sulfoamidierung erfolgen im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Tetrahydrofuran. Ebenso ist es möglich, Gemische der Lösemittel einzusetzen. Besonders bevorzugt sind Dichlormethan und Tetrahydrofuran.

Als Basen für die Amidierung und die Sulfoamidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.-buylat, oder organische Amine wie Benzyltrimethylammoniumhydroxid, Tetrabutylammoniumhydroxid, Pyridin, Triethylamin oder N-Methylpiperidin.

Die Amidierung und die Sulfoamidierung werden im allgemeinen in einem Temperaturbereich von 0°C bis 150°C, bevorzugt bei 25°C bis 40°C, durchgeführt.

Die Amidierung und die Sulfoamidierung werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Amidierung und der Sulfoamidierung wird die Base im allgemeinen in einer Menge von 1 bis 3 Mol, bevorzugt von 1 bis 1,5 Mol, bezogen auf 1 Mol der jeweiligen Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder 4-Dimethylaminopyridin.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Die Veresterung erfolgt im allgemeinen mit den entsprechenden Alkoholen in Anwesenheit von Säuren, vorzugsweise Schwefelsäure, in einem Temperaturbereich von 0°C bis 150°C, vorzugsweise von 50°C bis 100°C und Normaldruck.

Die Verbindungen der allgemeinen Formeln (IV) und (VIII) sind bekannt oder können nach üblichen Methoden hergestellt werden.

Die Verbindungen der allgemeinen Formel (VII) sind größtenteils neu und können beispielsweise wie oben beschrieben hergestellt werden.

Die Verbindungen der allgemeinen Formel (II) sind teilweise bekannt oder neu und können dann beispielsweise hergestellt werden, indem man die entsprechenden Amine mit Chlorameisensäuretrichlormethylester in einem der oben aufgeführten Lösemittel, vorzugsweise Xylol bei Rückflußtemperatur umsetzt.

Die Verbindungen der allgemeinen Formel (III) sind teilweise bekannt oder neu und können dann beispielsweise hergestellt werden, indem man ausgehend von den entsprechenen Carbonsäuren entweder mit Chlorameisensäureisobutylester / Aceton, Natriumazid/Wasser oder mit Diphenylphosphorylazid / Tetrahydrofuran oder mit Xylol oder Methylenchlorid in Gegenwart einer der oben angegebenen Basen, vorzugsweise Triethylamin, bei -10°C bis Raumtemperatur umsetzt.

Die Verbindungen der allgemeinen Formel (V) und (Va) sind teilweise bekannt oder neu und können entweder durch Abspaltung von Stickstoff aus den entsprechenden Carbonsäureaziden und Umsetzung mit den entsprechenden Alkoholen oder durch Umsetzung der entsprechenden Amine mit Chlorameisensäureestern, vorzugsweise Chlorameisensäurebenzylester in einem der oben aufgeführten Lösemittel, vorzugsweise Tetrahydrofuran oder Dioxan, in einem Temperaturbereich von -10°C bis 200°C, vorzugsweise von 0°C bis 150°C, hergestellt werden.

Die Verbindungen der allgemeinen Formel (Ia) sind neu und können beispielsweise wie unter [A], [B], [D] oder [E] beschrieben hergestellt werden.

Die Verbindungen der allgemeinen Formeln (Ib), (Ic), (Id) und (Ie) sind neu und können wie oben beschrieben hergestellt werden.

Die Verbindungen der allgemeinen Formel (VI) sind größtenteils bekannt oder neu und können beispielsweise hergestellt werden, indem man ausgehend von den freien Aminen (Ia) entweder mit dem Acetonid von Glycerinaldehyd in Methanol und in Anwesenheit von Natriumacetat / Natriumcyanborhydrid oder von Natriumboranat und Methanol in einem Temperaturbereich von -20°C bis +40°C, bevorzugt von -10°C bis 20°C und Normaldruck umsetzt.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

| **MHK-Werte (µg/ml):** | | | | | | | |
|---|---|---|---|---|---|---|---|
| **Bsp.- Nr.** | **Staph. 133** | **Staph. 48N** | **Staph. 25701** | **Staph. 9TV** | **E. coli Neumann** | **Klebs. 57 USA** | **Psdm. Bonn** |
| 12 | 2 | 2 | 2 | 2 | >64 | >64 | >64 |
| 13 | 8 | 8 | 8 | 8 | >64 | >64 | >64 |
| 16 | 4 | 4 | 4 | 4 | >64 | >64 | >64 |
| 18 | 4 | 4 | 2 | 2 | >64 | >64 | >64 |
| 19 | 1 | 1 | 1 | 0,25 | >64 | >64 | >64 |

Für schnellwachsende Mykobakterien wurde die MHK-Bestimmung in Anlehnung an die von Swenson beschriebene Methode der Bouillon-Mikrodilution durchgeführt [vgl. J.M. Swenson, C. Thornberry, U.A. Silcox, Rapidly growing mycobacteria. Testing of susceptibility to 34 antimicrobial agents by broth microdilution. Antimicrobial Agents and Chemotherapy Vol, 22, 186-192 (1982)]. Abweichend davon war das mit 0,1 Vol.% Tween 80 versetzte Hirn-Herzextrakt Medium.

Die verwendeten Mykobakterienstämme wurden von der DSM (Dt. Sammlung von Mikroorganismen, Braunschweig) bezogen. Sie wurden in einer feuchten Kammer bei 37°C bebrütet.

Die MHK-Werte wurden nach 2-4 Tagen abgelesen, wenn die präparatfreien Kontrollen durch Wachstum trüb waren. Der MHK-Wert definiert sich als die niedrigste Präparatkonzentration, die makroskopisch sichtbares Wachstum völlig inhibiert.

| **MHK Werte (µg/ml): Mycobacterium smegmatis** | | |
|---|---|---|
| **Stamm:** | **DSM 43061** | **DSM 43465** |
| Bsp.-Nr. | | |
| 13 | 16 | 8 |
| 19 | 32 | 16 |
| Isoniazid | 4 | 1 |
| Streptomycin | 4 | 4 |

Die erfindungsgemäßen Verbindungen der allgemeinen Formeln (I), (Ia), (Ib), (Ic), (Id) und (Ie) weisen bei geringer Toxizität ein breites antibakterielles Spektrum, speziell gegen gram-positive Bakterien, Haemophilus influenzae, anaerobe Keime und für schnellwachsende Mykobakterien auf. Diese Eigenschaften ermöglichen ihre Verwendung als chemotherapeutische Wirkstoffe in der Human- und Tiermedizin.

Besonders wirksam sind die erfindungsgemäßen Verbindungen gegen Bakterien und bakterienännliche Mikroorganismen wie Mycoplasmen. Sie sind daher besonders gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die erfindungsgemäßen Verbindungen können zum Zweck der Erweiterung des Wirkungsspektrums und um eine Wirkungssteigerung zu erreichen auch mit anderen Antibiotika kombiniert werden.

### Anhang zum experimentellen Teil

### Liste der verwendeten Laufmittelgemische zur Chromatographie:

I Dichlormethan : Methanol
II Toluol : Ethylacetat
III Acetonitril : Wasser
IV Ethylacetat
V Petrolether : Ethylacetat

### Abkürzungen:

- Z: Benzyloxycarbonyl
- Boc: tert.Butyloxycarbonyl
- DMF: Dimethylformamid
- Ph: Phenyl
- Me: Methyl
- THF: Tetrahydrofuran
- CDI: Carbonyldiimidazol
- DCE: Dichlorethan

### Ausgangsverbindungen

### Beispiel I

### 3-Amino-6-methyl-thieno[2,3-b]pyridin-2-carbonsäuremethylester

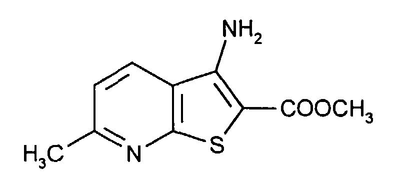

45 g (295 mmol)) 2-Chlor-6-methylpyridin-3-carbonitril werden in 180 ml DMSO gelöst, mit 90 ml (649 mmol) Triethylamin und 28 ml (310 mmol) Mercaptoessigsäuremethylester versetzt und 18 h bei 80°C verrührt. Man läßt auf Raumtemperatur kommen, kippt auf Eiswasser, saugt ab, wäscht den Rückstand mit Petrolether nach und trocknet 5 h im Umlufttrockenofen bei 60°C.
Ausbeute: 63 g (96%)
MS: 222 [M⁺, 100%]
¹H-NMR (D₆-DMSO, TMS): 8,4 (d, J = 9 Hz, 1H); 7,83 (d, J = 9 Hz, 1H); 7,26 (s, 2H); 3,8 (s, 3H); 2,58 (s, 3H).

### Beispiel II

### 3-Amino-5-methyl-thieno[2,3-b]pyridin-2-carbonsäuremethylester

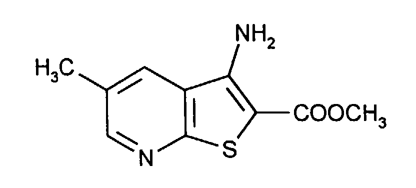

37,5 g (250 mmol) 2-Mercapto-3-cyano-5-methylpyridin werden in 175 ml DMSO gelöst und mit 76 ml (550 mmol) Triethylamin versetzt. Zu der so erhaltenen Lösung tropft man innerhalb von 5 min 22 ml (250 mmol) Chloressigsäuremethylester zu. Man verrührt 5 h bei 80°C, gibt auf Eiswasser, saugt vom ausgefallenen Feststoff ab, wäscht diesen mit Diethylether gut nach und trocknet im Umlufttrockenofen bei 50°C.
Ausbeute: 53,5 g (96%)
MS: 222 [M⁺, 100%]
¹H-NMR (D₆-DMSO): 8,55 (s, 1H); 8,35 (s, 1H); 7,25 (s, 2H); 3,7 (s, 3H); 2,4 (s, 3H).

### Beispiel III

### 6-Methyl-thieno[2,3-b]pyridin-2-carbonsäuremethylester

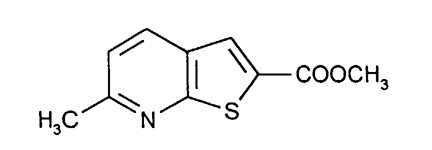

209 ml Wasser werden vorsichtig mit 628 ml H₂SO₄ konz. versetzt, auf 0°C gekühlt und mit 62 g (279 mmol) der Verbindung aus Beispiel I versetzt. Nun wird eine Lösung von 61,5 g (894 mmol) Natriumnitrit in 280 ml Wasser so zugetropft, daß die Innentemperatur der Reaktionslösung +5°C nicht übersteigt. Nach beendeter Zugabe wird 1 h bei 0°C nachgerührt. Die so erhaltene Reaktionslösung wird so in 1,675 l 50%ige Hypophosphorsäure eingetragen, daß die Innentemperatur nicht über +7°C ansteigt. Nach beendeter Zugabe läßt man 30 min bei 0°C nachrühren und hält über Nacht bei +4°C. Nun wird mit festem NaHCO₃ neutral gestellt (schäumt heftig) und vom ausgefallenen Feststoff abgesaugt. Der Rückstand wird in 2 l Aceton 10 min verrührt, abgesaugt und im Umlufttrockenofen bei 50°C getrocknet.
Ausbeute: 24,3 g (42%)
MS: 207 [M⁺, 90%]
¹H-NMR (D₆-DMSO, TMS): 8,3 (d, J = 9 Hz, 1H); 8,15 (s, 1H); 7,4 (d, J = 9 Hz, 1H); 3,9 (d, 3H); 2,63 (s, 3H).

### Beispiel IV

### 6-Methyl-thieno[2,3-b]pyridin-2-carbonsäure

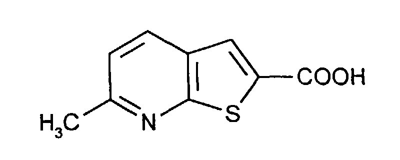

23 g (111 mmol) der Verbindung aus Beispiel III werden in 660 ml Ethanol gelöst, mit 93,5 g (1,66 mol) Kaliumhydroxid versetzt und 30 min am Rückfluß gekocht. Nach Abkühlen auf Raumtemperatur wird vom Niederschlag abgesaugt und dieser gut mit Ethanol nachgewaschen. Der Niederschlag wird in Wasser gelöst und mit Essigsäure auf pH 4 angesäuert. Von der ausgefallenen Säure wird abgesaugt, mit 2 l Petrolether nachgewaschen und im Umlufttrockenschrank bei 50°C getrocknet.
Ausbeute: 18,6 g (87%)
¹H-NMR (D₆-DMSO, TMS): 12,1 (s, 1H); 8,28 (d, J = 9 Hz, 1H); 8,05 (s, 1H); 7,39 (d, J = 9 Hz, 1H); 2,62 (s, 3H).

Analog den Vorschriften der Verbindungen I - IV werden die in der Tabelle I aufgeführten Verbindungen dargestellt:

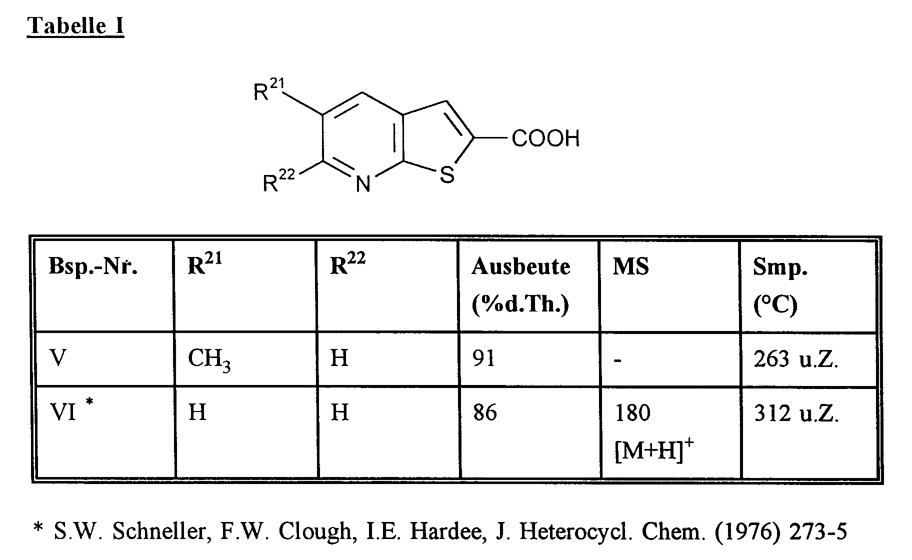

### Beispiel VII

### 6-Methyl-thieno[2,3-b]pyridin-2-carbonsäureazid

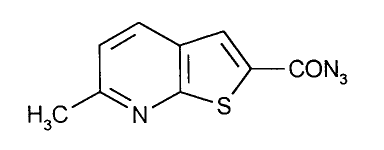

18 g (93,2 mmol) der Verbindung aus Beispiel IV werden in 180 ml Aceton gelöst und mit 15,4 ml (110 mmol) Triethylamin versetzt. Diese Reaktionsmischung wird auf -15°C gekühlt und langsam mit einer Lösung von 15,4 ml (121 mmol) Chlorameisensäureisobutylester in 77 ml Aceton versetzt, so daß die Innentemperatur -5°C nicht übersteigt. Man rührt 2 h bei -10°C nach und tropft eine Lösung von 9 g (140 mmol) Natriumazid in Wasser zu, rührt 2 h bei 0°C nach, kippt auf 2,5 l Eiswasser, saugt vom ausgefallenen Niederschlag ab, wäscht diesen mit Wasser gut nach und trocknet an der Luft.
Ausbeute: 18 g (89% d.Th.)

### Beispiel VIII

### 2-Butyloxycarbonylamino-6-methyl-thieno[2,3-b]pyridin

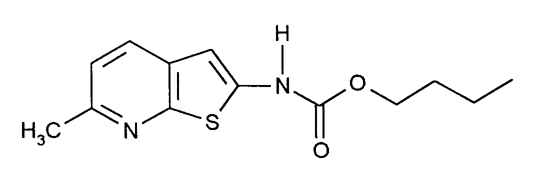

18 g (82 mmol) der Verbindung aus Beispiel VII werden portionsweise in 390 ml siedendes Butanol eingetragen. Nach beendeter Zugabe wird 10 min unter Rückfluß nachgerührt, auf Raumtemperatur abgekühlt, eingeengt, in Diethylether verrührt, abgesaugt und im Umlufttrockenofen bei 50°C getrocknet.
Ausbeute: 20,3 g (93%)
Smp.: 162°C
¹H-NMR (D₆-DMSO, TMS): 7,88 (d, J = 9 Hz, 1H); 7,24 (d, J = 9 Hz, 1H); 6,75 (s, 1H); 4,18 (t, J = 7 Hz, 2H); 2,53 (s, 3H); 1,65 (q, J = 7 Hz, 2H); 1,39 (h, J = 7 Hz, 2H); 0,93 (t, J = 7 Hz, 3H).

Analog den Vorschriften der Verbindungen VII und VIII werden die in der Tabelle II aufgeführten Verbindungen dargestellt:

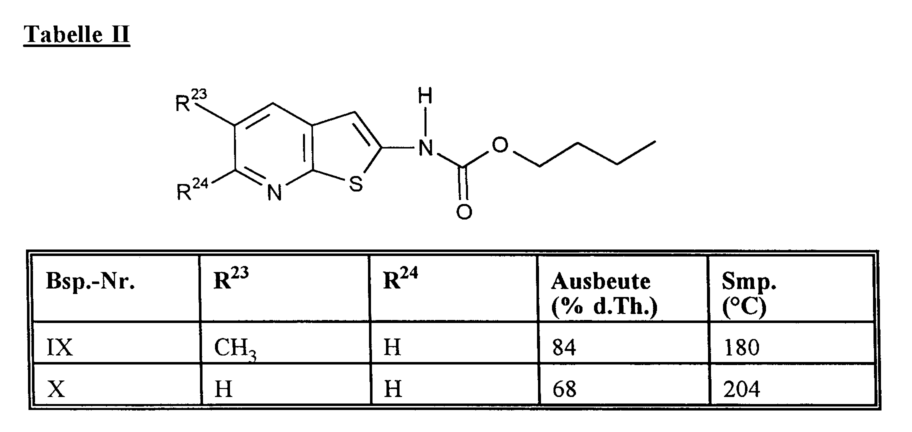

### Herstellungsbeispiele

### Beispiel 1

### (5R)-3-[6-Methyl-pyrido[2,3-b]thienyl]-5-hydroxymethyl-oxazolidin-2-on

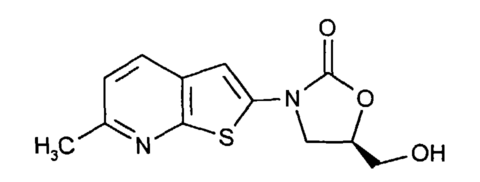

20,3 g (76,8 mmol) der Verbindung aus Beispiel VIII werden in 150 ml THF gelöst, mit 10 mg Benzylidenbenzylimin versetzt und auf -70°C gekühlt. Nun werden langsam ca. 31 ml 2,5 n-Butyllithium.-Lösung in Hexan bis zum Farbumschlag nach rot zugetropft. Anschließend werden 10,9 ml (76,8 mmol) (R)-Glycidylbutyrat zugetropft. Man läßt auf Raumtemperatur kommen, versetzt mit gesättigter Ammoniumchlorid-Lösung, rührt 30 min bei Raumtemperatur nach und saugt vom ausgefallenen Niederschlag ab. Der Rückstand wird mit wenig Wasser und mit viel Diethylether gewaschen und im Umluftttrockenofen bei 50°C getrocknet.
Ausbeute: 19,7 g (97% d.Th.)
Smp.: 245°C u.Z.
R_{f}: 0,24 (I, 100:5)
MS: 265 [(M+H)⁺, 100%]
¹H-NMR (D₆-DMSO, TMS): 7,95 (d, J = 9 Hz, 1H); 7,25 (d, J= 9 Hz, 1H); 6,69 (s, 1H); 5,3 (s, 1H); 4,8 - 4,96 (m, 1H); 4,18 (t, J = 9,5 Hz, 1H); 3,93 (dd, J = 9,5 Hz, 6,5 Hz, 1H); 3,55 - 3,8 (m, 2H); 2,55 (s, 3H).

Analog Verbindung 1 wurden die in der Tabelle 1 aufgeführten Verbindungen dargestellt:

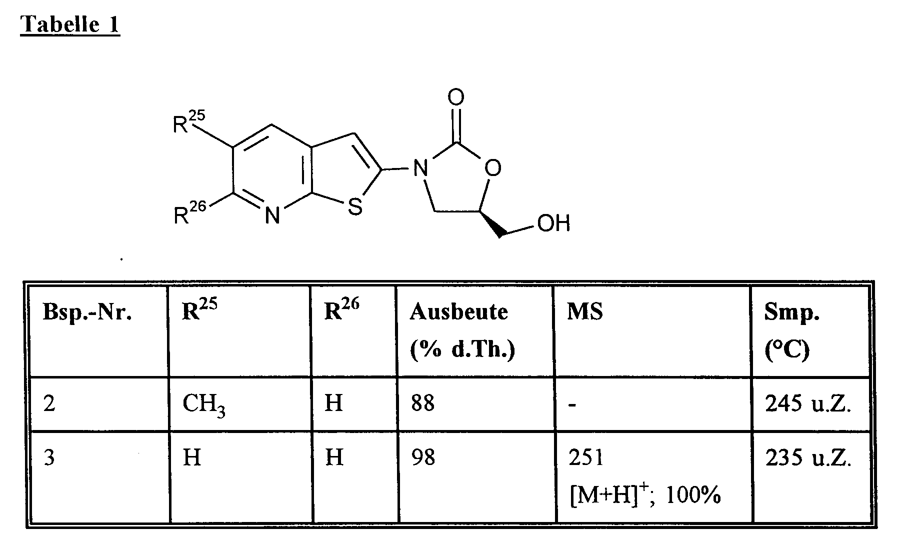

### Beispiel 4

### (5R)-3-[6-Methyl-pyrido[2,3-b]thienyl]-5-methansulfonyloxymethyl-oxazolidin-2-on

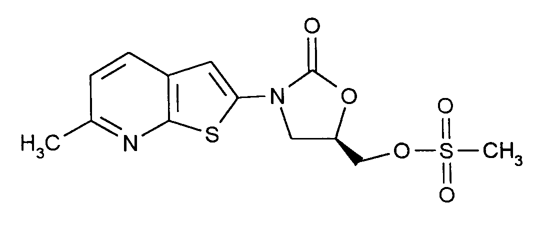

Eine Lösung von 18,8 g (71 mmol) der Verbindung aus Beispiel 1 in 290 ml Pyridin wird auf 0°C gekühlt und langsam mit 11 ml (142 mmol) Methansulfonsäurechlorid versetzt. Es wird 16 h bei 4°C gehalten und eingeengt. Der Rückstand wird in 5%iger Natriumhydrogencarbonatlösung verrührt, abgesaugt und mit Wasser und Diethylether nachgewaschen und im Umlufttrockenofen bei 50°C getrocknet.
Ausbeute: 23 g (95% d.Th.)
R_{f} = 0,47 (I, 100:5)

### Beispiel 5

### (5R)-3-[6-Methyl-pyrido[2,3-b]thienyl]-5-azido-methyl-oxazolidin-2-on

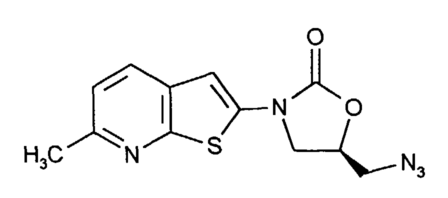

23 g (67,1 mmol) der Verbindung aus Beispiel 4 werden in 160 ml DMF gelöst und mit 4,8 g (74 mmol) Natriumazid versetzt. Die so erhaltene Reaktionsmischung wird 16 h bei 70°C verrührt. Man läßt auf Raumtemperatur abkühlen und kippt auf 2 l Eiswasser. Man saugt vom ausgefallenen Feststoff ab, wäscht mit Wasser und Petrolether nach und trocknet an der Luft.
Ausbeute: 17,9 g (92% d.Th.)
R_{f}: 0,31 (I, 100:2)
Smp.: 181°C u.Z.
MS: 290 [(M+H)⁺; 100%]
¹H-NMR (D₆-DMSO, TMS): 7,96 (d, J ) 9 Hz, 1H); 7,75 (d, J = 9 Hz, 1H); 6,72 (s, 1H); 4,98 - 5,12 (m, 1H); 4,24 (t, J = 9,5 Hz, 1H); 3,78 - 3,9 (m, 3H); 2,55 (s, 3H).

Analog den Vorschriften der Beispiele 4 und 5 werden die in der Tabelle 2 aufgeführten Verbindungen dargestellt:

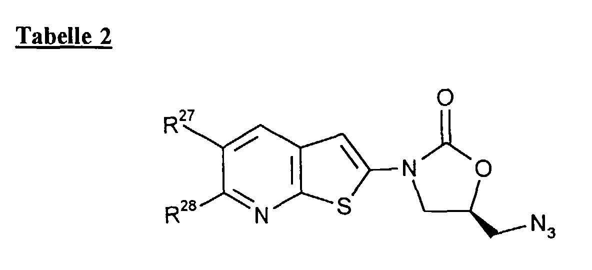

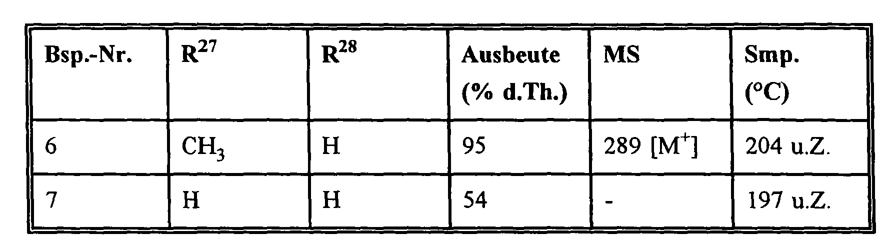

### Beispiel 8

### (5S)-3-[6-Methyl-pyrido[2,3-b]thienyl]-5-aminomethyl-oxazolidin-2-on Hydrochlorid

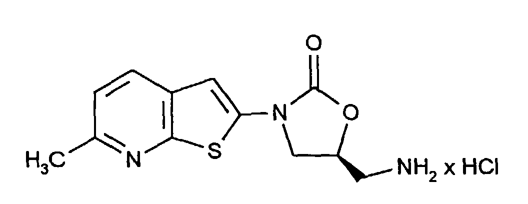

5 g (17,3 mmol) der Verbindung aus Beispiel 5 werden in 400 ml Ethanol gelöst, mit 500 mg 5%igem Palladium auf Aktivkohle versetzt und 16 h unter 3 bar Wasserstoffdruck hydriert. Man filtriert vom Katalysator ab, engt ein, nimmt in Methylenchlorid auf und versetzt langsam mit 5 ml 4,5 N HCl in Ether. Man rührt 1 h bei Raumtemperatur nach, saugt ab und wäscht mit Ether nach. Der Rückstand wird bei 40°C im Umlufttrockenofen getrocknet.
Ausbeute: 5,74 g (98% d.Th.)
¹H-NMR (D₂O): 8,3 (d, J = 9 Hz, 1H); 7,5 (d, J = 9 Hz, 1H); 6,78 (s, 1H); 5,11 - 5,27 (m, 1H); 4,37 (t, J = 9,5 Hz, 1H); 3,95 (dd, J = 9,5 Hz, J = 6,5 Hz, 1H); 3,30 - 3,5 (m, 2H); 2,65 (s, 3H).

Analog der Verbindung 8 werden die in der Tabelle 3 aufgeführten Verbindungen dargestellt:

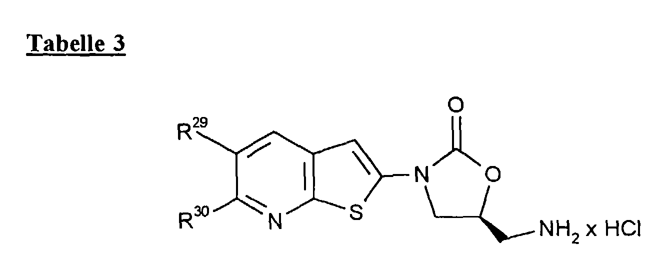

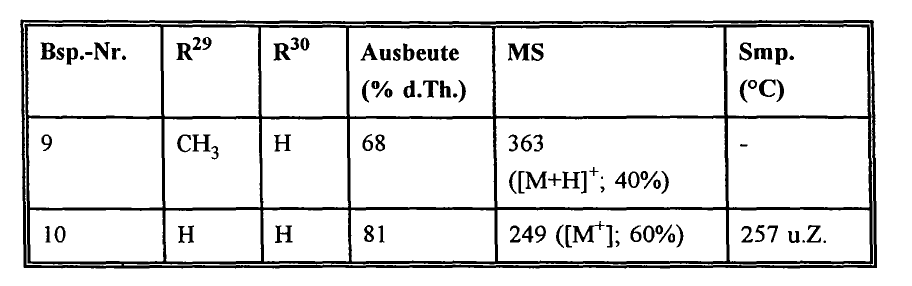

### Beispiel 11

### (5S)-3-[6-Methyl-pyrido[2,3-b]thienyl]-5-acetylaminomethyl-oxazolidin-2-on

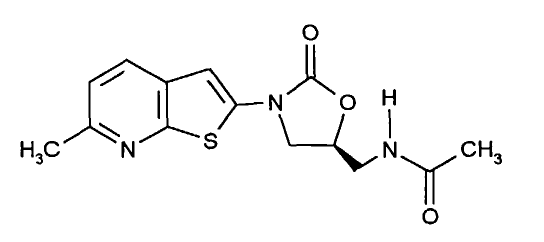

1,5 g (4,1 mmol) der Verbindung aus Beispiel 8 werden mit 1,14 ml (8,2 mmol) Triethylamin versetzt und in 8 ml Pyridin gelöst. Man kühlt die Reaktionslösung auf 0°C ab und tropft 0,73 ml (10,2 mmol) Acetylchlorid zu. Nach 4 Stunden bei 0°C wird mit 1 ml Methanol versetzt, eingeengt und an Kieselgel (Methlyenchlorid : Methanol = 100:3) chromatographiert.
Ausbeute: 0,84 g (67%)
Smp.: 215°C u.Z.
R_{f}: 0,44 (I; 10:1)
MS: 306 [(M+H)⁺; 100%]
¹H-NMR (D₆-DMSO, TMS) 8,3 (t, J = 6,5 Hz, 1H); 7,95 (d, J= 9 H, 1H); 7,25 (d, J = 9 Hz, 1H); 6,68 (s, 1H); 4,83 - 4,98 (m, 1H); 4,2 (t, J = 9,5 Hz, 1H); 3,83 (dd, J = 9,5 Hz, J = 6,5 Hz, 1H); 3,47 (t, J = 6 Hz, 2H); 2,55 (s, 3H); 1,85 (s, 3H).

### Beispiel 12

### (5S)-3-[6-Methyl-pyrido[2,3-b]thienyl]-5-thioacetylaminomethyl-oxazolidin-2-on

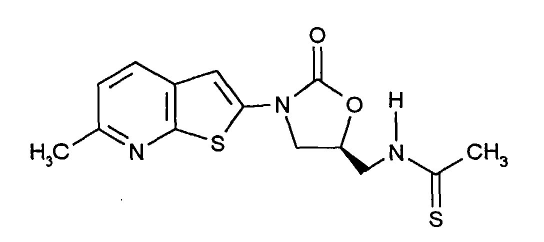

673 mg (2 mmol) der Verbindung aus Beispiel 8 werden in 4 ml THF gelöst, mit 0,61 ml (4,4 mmol) Triethylamin und 0,26 ml (2,2 mmol) Ethyldithioacetat versetzt und 18 h bei Raumtemperatur gerührt. Man engt ein und chromatographiert an Kieselgel (Methylenchlorid : Methanol = 100:1).
Ausbeute: 475 mg (74%)
Smp.: 202 u.Z.
R_{f}: 0,3 (I; 100:5)
MS: 321 (M⁺, 20%)
¹H-NMR (D₆-DMSO, TMS): 10,45 (s, 1H); 7,95 (d, J = 9 Hz, 1H); 7,25 (d, J = 9 Hz, 1H); 6,68 (s, 1H); 5,05 - 5,2 (m, 1H); 4,25 (t, J = 9,5 Hz, 1H); 3,98 (t, J = 6,5 Hz, 2H); 3,9 (dd, J = 9,5 Hz, J = 6,5 Hz, 1H); 2,55 (s 3H); 2,43 (s, 3H).

Analog den Vorschriften der Beispiele 11 und 12 wurden die in Tabelle 4 aufgeführten Verbindungen dargestellt:

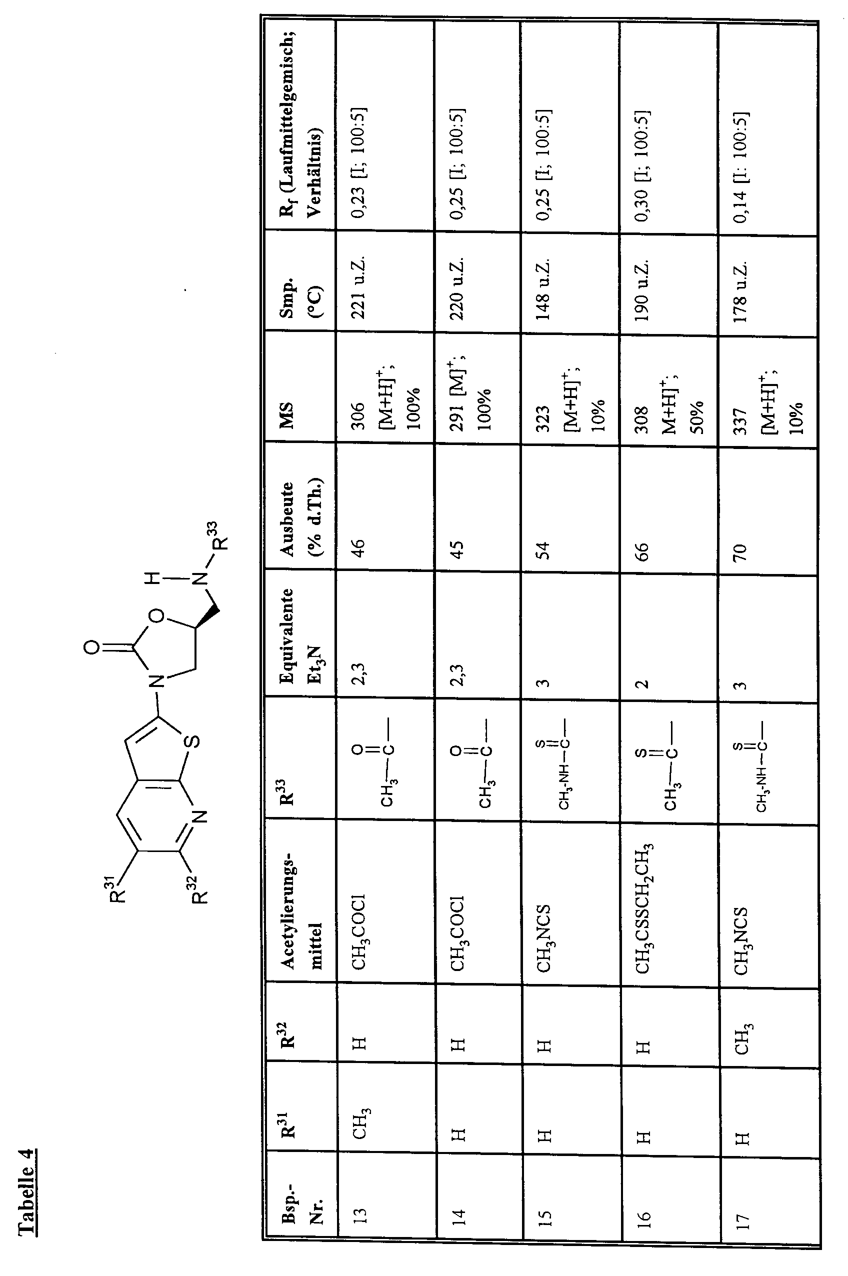

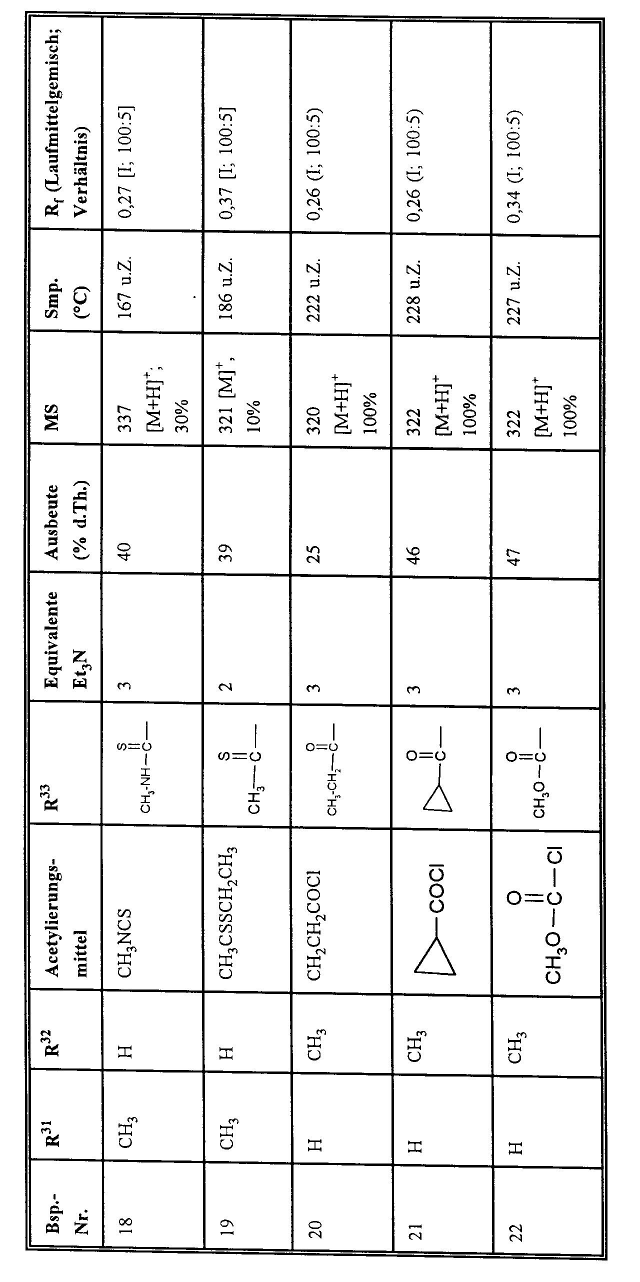

## Patentansprüche

1. Verbindungen der allgemeinen Formel (I) 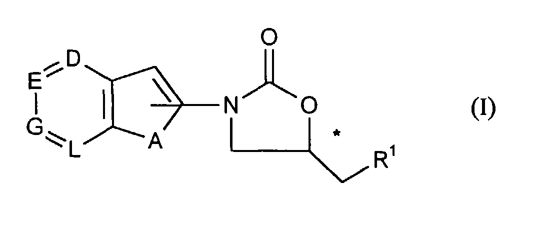 in welcher
A für ein Sauerstoff- oder Schwefelatom oder für die SO₂-Gruppe steht,
und
D, E, G und L gleich oder verschieden sind und mindestens einer dieser Substituenten für ein Stickstoffatom steht und die übrigen für einen Rest der Formel -CR² stehen,
worin
R² Wasserstoff, Cyano, Nitro, Carboxyl, geradkettiges oder verzweigtes Alkyl, Acyl oder Alkoxy mit jeweils bis zu 7 Kohlenstoffatomen, Halogen oder eine Gruppe der Formel - NR³R⁴, -CO-NR⁵R⁶, -NR⁷-CO-R⁸ oder -S(O)ₐR⁹ bedeutet,
worin
R³, R⁴, R⁵, R⁶, R⁷ und R⁸ gleich oder verschieden sind und Wasserstoff, Phenyl oder geradkettiges oder verzweigtes Alkyl mit bis zu 6 Kohlenstoffatomen bedeuten,
a eine Zahl 0, 1 oder 2 bedeutet,
R⁹ Phenyl oder geradkettiges oder verzweigtes Alkyl mit bis zu 4 Kohlenstoffatomen bedeutet,
R¹ für Azido, Hydroxy oder für eine Gruppe der Formel -OR¹⁰, O-SO₂R¹¹ oder -NR¹²R¹³ steht,
worin
R¹⁰ geradkettiges oder verzweigtes Acyl mit bis zu 8 Kohlenstoffatomen oder eine Hydroxyschutzgruppe bedeutet,
R¹¹ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,
R¹² und R¹³ gleich oder verschieden sind und Cycloalkyl mit 3 bis 6 Kohlenstoffatomen, Wasserstoff, Phenyl oder geradkettiges oder verzweigtes Alkyl oder Alkoxy mit jeweils bis zu 8 Kohlenstoffatomen oder eine Aminoschutzgruppe bedeuten,
oder
R¹² oder R¹³ eine Gruppe der Formel -CO-R¹⁴, -CS-R^{14'}, P(O)(OR¹⁵)(OR¹⁶) oder -SO₂-R¹⁷ bedeutet,
worin
R¹⁴ und R^{14'} gleich oder verschieden sind und Cycloalkyl mit 3 bis 6 Kohlenstoffatomen, Trifluormethyl, geradkettiges oder verzweigtes Alkoxy mit bis zu 8 Kohlenstoffatomen, Phenyl, Benzyloxy oder Wasserstoff bedeuten, oder
R¹⁴ und R^{14'} geradkettiges oder verzweigtes Alkyl mit bis zu 8 Kohlenstoffatomen bedeuten, das gegebenenfalls durch Cyano, Halogen oder Trifluormethyl substituiert ist,
oder
geradkettiges oder verzweigtes Thioalkyl oder Acyl mit jeweils bis zu 6 Kohlenstoffatomen bedeuten,
oder
eine Gruppe der Formel -NR¹⁸R¹⁹ bedeuten,
worin
R¹⁸ und R¹⁹ gleich oder verschieden sind und Wasserstoff, Phenyl oder geradkettiges oder verzweigtes Alkyl mit bis zu 6 Kohlenstoffatomen bedeuten,
oder
einen 5-gliedrigen aromatischen Heterocyclus mit bis zu 3 Heteroatomen aus der Reihe S, N und/oder O bedeuten,
R¹⁵ und R¹⁶ gleich oder verschieden sind und Wasserstoff oder geradkettiges oder verzweigtes Alkyl mit bis zu 4 Kohlenstoffatomen bedeuten,
R¹⁷ geradkettiges oder verzweigtes Alkyl mit bis zu 4 Kohlenstoffatomen oder Phenyl bedeutet
als reine Stereoisomere oder als Stereoisomerengemisch,
und deren Salze.

2. Verbindungen der allgemeinen Formel (I) gemäß Anspruch 1, in welcher
A für ein Sauerstoff- oder Schwefelatom oder für die -SO₂-Gruppe steht,
und
D, E, G und L gleich oder verschieden sind und mindestens einer dieser Substituenten für ein Stickstoffatom steht und die übrigen für einen Rest der Formel -CR² stehen,
worin
R² Wasserstoff, geradkettiges oder verzweigtes Alkyl mit bis zu 6 Kohlenstoffatomen, Fluor, Chlor oder Brom bedeutet,
R¹ für Azido, Hydroxy oder für eine Gruppe der Formel -OR¹⁰, O-SO₂R¹¹ oder -NR¹²R¹³ steht,
worin
R¹⁰ geradkettiges oder verzweigtes Acyl mit bis zu 6 Kohlenstoffatomen oder Benzyl bedeutet,
R¹¹ geradkettiges oder verzweigtes Alkyl mit bis zu 3 Kohlenstoffatomen, Phenyl oder Tolyl bedeutet,
R¹² und R¹³ gleich oder verschieden sind und Cyclopropyl, Cyclopentyl, Cyclohexyl, Wasserstoff, Phenyl oder geradkettiges oder verzweigtes Alkyl oder Alkoxy mit jeweils bis zu 6 Kohlenstoffatomen, tert.Butoxycarbonyl oder Benzyloxycarbonyl bedeuten,
oder
R¹² oder R¹³ eine Gruppe der Formel -CO-R¹⁴, -CS-R^{14'}, P(O)(OR¹⁵)(OR¹⁶) oder -SO₂-R¹⁷ bedeutet,
worin
R¹⁴ und R^{14'} gleich oder verschieden sind und Cyclopropyl, Cyclobutyl, Cyclopentyl, Cyclohexyl, Trifluormethyl oder geradkettiges oder verzweigtes Alkoxy mit bis zu 6 Kohlenstoffatomen, Phenyl, Benzyloxy oder Wasserstoff bedeuten, oder
R¹⁴ und R^{14'} geradkettiges oder verzweigtes Alkyl mit bis zu 6 Kohlenstoffatomen bedeuten, das gegebenenfalls durch Cyano, Fluor, Chlor, Brom oder Trifluormethyl substituiert ist, oder
geradkettiges oder verzweigtes Thioalkyl oder Acyl mit jeweils bis zu 5 Kohlenstoffatomen bedeuten, oder
eine Gruppe der Formel -NR¹⁸R¹⁹ bedeuten,
worin
R¹⁸ und R¹⁹ gleich oder verschieden sind und Wasserstoff, Phenyl oder geradkettiges oder verzweigtes Alkyl mit bis zu 4 Kohlenstoffatomen bedeuten, oder
oder
Isoxazolyl, Furyl, Thienyl, Pyrryl, Oxazolyl oder Imidazolyl bedeuten,
R¹⁵ und R¹⁶ gleich oder verschieden sind und Wasserstoff oder geradkettiges oder verzweigtes Alkyl mit bis zu 3 Kohlenstoffatomen bedeuten,
R¹⁷ geradkettiges oder verzweigtes Alkyl mit bis zu 3 Kohlenstoffatomen oder Phenyl bedeutet
als reine Stereoisomere oder als Stereoisomerengemisch,
und deren Salze.

3. Verbindungen der allgemeinen Formel (I) gemäß Anspruch 1, in welcher
A für ein Sauerstoff- oder Schwefelatom oder für die -SO₂-Gruppe steht,
und
D, E, G und L gleich oder verschieden sind und für mindestens ein Stickstoffatom oder für den Rest der Formel -CR² stehen,
worin
R² Wasserstoff, geradkettiges oder verzweigtes Alkyl mit bis zu 3 Kohlenstoffatomen oder Fluor bedeutet,
R¹ für Azido, Hydroxy oder für eine Gruppe der Formel -OR¹⁰, O-SO₂R¹¹ oder -NR¹²R¹³ steht,
worin
R¹⁰ geradkettiges oder verzweigtes Acyl mit bis zu 5 Kohlenstoffatomen oder Benzyl bedeutet,
R¹¹ Methyl, Ethyl, Phenyl oder Tolyl bedeutet,
R¹² und R¹³ gleich oder verschieden sind und Cyclopropyl, Cyclopentyl, Cyclohexyl, Wasserstoff, Phenyl oder geradkettiges oder verzweigtes Alkyl oder Alkoxy mit jeweils bis zu 5 Kohlenstoffatomen, tert.Butoxycarbonyl oder Benzyloxycarbonyl bedeuten,
oder
R¹² oder R¹³ eine Gruppe der Formel -CO-R¹⁴, -CS-R^{14'}, P(O)(OR¹⁵)(OR¹⁶) oder -SO₂R¹⁷ bedeutet,
worin
R¹⁴ und R^{14'} gleich oder verschieden sind und Cyclopropyl, Cyclobutyl, Cyclopentyl, Cyclohexyl, Trifluorethyl oder geradkettiges oder verzweigtes Alkoxy mit bis zu 5 Kohlenstoffatomen, Phenyl, Benzyloxy oder Wasserstoff bedeuten,
oder
R¹⁴ und R^{14'} geradkettiges oder verzweigtes Alkyl mit bis zu 5 Kohlenstoffatomen bedeuten, das gegebenenfalls durch Cyano, Fluor, Chlor, Brom oder Trifluormethyl substituiert ist, oder
geradkettiges oder verzweigtes Thioalkyl oder Acyl mit jeweils bis zu 4 Kohlenstoffatomen bedeuten, oder
eine Gruppe der Formel -NR¹⁸R¹⁹ bedeuten,
worin
R¹⁸ und R¹⁹ gleich oder verschieden sind und Wasserstoff, Phenyl oder geradkettiges oder verzweigtes Alkyl mit bis zu 3 Kohlenstoffatomen bedeuten, oder Isoxazolyl, Furyl, Oxazolyl oder Imidazolyl bedeuten,
R¹⁵ und R¹⁶ gleich oder verschieden sind und Wasserstoff, Methyl oder Ethyl bedeuten,
R¹⁷ Methyl oder Phenyl bedeutet
als reine Stereoisomeren oder als Stereoisomerengemisch,
und deren Salze.

4. Verbindungen der allgemeinen Formel (I) gemäß Anspruch 1, in welcher der Oxazolidinonrest in der Position 2 am 5-Ring-Heterocyclus angebunden ist, als reine Stereoisomere oder als Stereoisomerengemisch, und deren Salze.

5. Verbindungen gemäß Anspruch 1, ausgewählt aus der Gruppe
(5S)-3-[6-Methyl-pyrido[2,3-b]thienyl]-5-thioacetylaminomethyl-oxazolidin-2-on,
(5S)-3-[5-Methyl-pyrido[2,3-b]thienyl]-5-acetylaminomethyl-oxazolidin-2-on,
(5S)-3-[Pyrido[2,3-b]thien-2-yl]-5-thioacetyl-aminomethyl-oxazolidin-2-on, 1-Methyl-3-(2-oxo-3-[5-(5S)-methyl-thieno-[2,3-b]pyridin-2-yl]-oxazolidin-5-ylmethyl)-thioharnstoff und
(5S)-3-[5-Methyl-pyrido[2,3-b]-thienyl]-5-thioacetylaminomethyl-oxazolidin-2-on.

6. Verfahren zur Herstellung von Verbindungen der allgemeinen Formel (I) gemäß Anspruch 1, dadurch gekennzeichnet, daß man
[A] Verbindungen der allgemeinen Formel (II) oder (III) 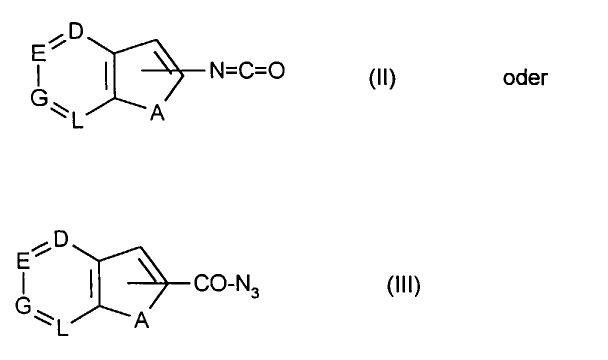 in welchen
A, D, E, G und L die in Anspruch 1 angegebenen Bedeutungen haben,
mit Lithiumbromid/(C₄H₉)₃ P(O) und Epoxiden der allgemeinen Formel (IV) 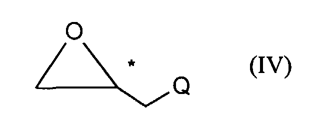 in welcher
Q für C₁-C₆-Acyloxy steht,
in inerten Lösemitteln, gegebenenfalls in Anwesenheit einer Base
umsetzt,
und im Fall R¹ = OH durch eine typische Esterverseifung oder durch eine typische Umesterung die Hydroxyfunktion freisetzt,
oder
[B] Verbindungen der allgemeinen Formel (V) 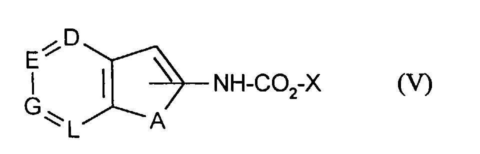 in welcher
A, D, E, G und L die oben angegebene Bedeutung haben
und
X für eine typische Schutzgruppe steht,
in inerten Lösemitteln und in Anwesenheit einer Base mit Epoxiden der allgemeinen Formel (IV) umsetzt,
oder
[C] im Fall R¹ = OH, zunächst Verbindungen der allgemeinen Formel (III) durch Abspaltung von Stickstoff in Alkoholen in die Verbindungen der allgemeinen Formel (Va) 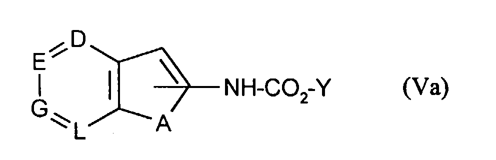 in welcher
A, D, E, G und L die oben angegebene Bedeutung haben
und
Y für geradkettiges oder verzweigtes C₂-C₆-Alkyl steht,
überführt,
und in einem zweiten Schritt wie unter [A] beschrieben in inerten Lösemitteln und in Anwesenheit einer Base mit Epoxiden der allgemeinen Formel (IV) umsetzt,
oder
[D] Verbindungen der allgemeinen Formel (VI) 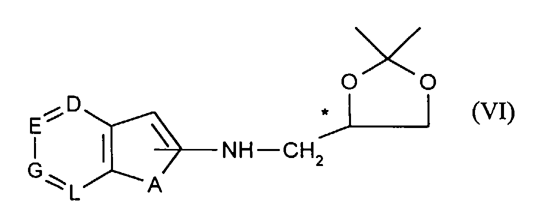 in welcher
A, D, E, G und L die oben angegebene Bedeutung haben,
entweder direkt mit Säuren und Kohlensäurediethylester umsetzt,
oder zunächst durch Umsetzung der Verbindungen der allgemeinen Formel (VI) mit Säuren die Verbindungen der allgemeinen Formel (VII) 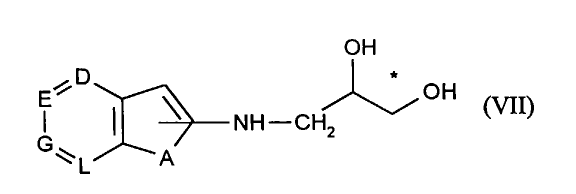 in welcher
A, D, E, G und L die oben angegebene Bedeutung haben,
herstellt,
und anschließend in Anwesenheit eines Hilfsmittels in inerten Lösemitteln cyclisiert,
oder
[E] zunächst Verbindungen der allgemeinen Formel (Ia) 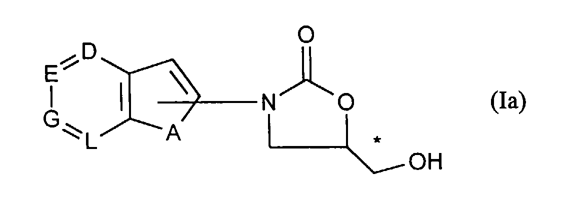 in welcher
A, D, E, G und L die oben angegebene Bedeutung haben,
durch Umsetzung mit (C₁-C₄)-Alkyl- oder Phenylsulfonsäurechloriden, die gegebenenfalls entsprechend substituiert sind, in inerten Lösemitteln und in Anwesenheit einer Base in die entsprechenden Verbindungen der allgemeinen Formel (Ib) 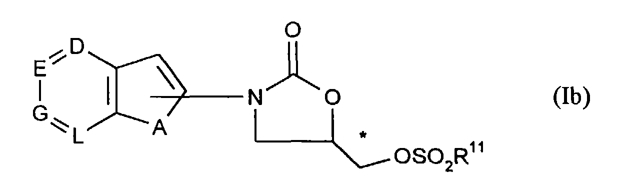 in welcher
A, D, E, G und L die oben angegebene Bedeutung haben und
R¹¹ die in Anspruch 1 angegebene Bedeutung hat,
überführt,
anschließend mit Natriumazid in inerten Lösemitteln die Azide der allgemeinen Formel (Ic) 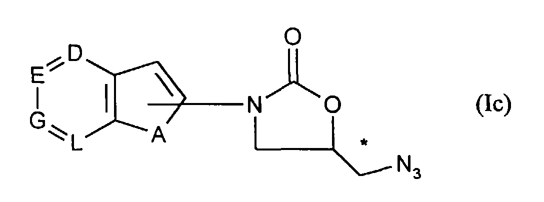 in welcher
A, D, E, G und L die oben angegebene Bedeutung haben,
herstellt,
in einem weiteren Schritt durch Umsetzung mit (C₁-C₄-O)₃-P oder PPh₃ in inerten Lösemitteln und mit Säuren in die Amine der allgemeinen Formel (Id) 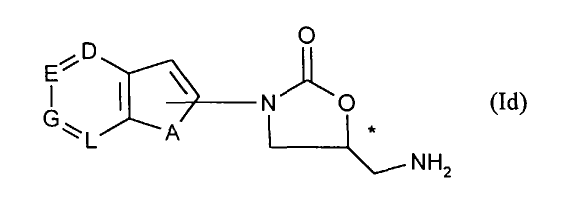 in welcher
A, D, E, G und L die oben angegebene Bedeutung haben,
überführt,
und durch Umsetzung mit Acetanhydrid oder anderen Acylierungsmitteln der allgemeinen Formel (VIII)
R²⁰-CO-R¹⁴ (VIII)
in welcher
R¹⁴ die in Anspruch 1 angegebene Bedeutung hat
und
R²⁰ für Halogen oder für den Rest -OCOR¹⁴ steht,
in inerten Lösemitteln die Verbindungen der allgemeinen Formel (Ie) 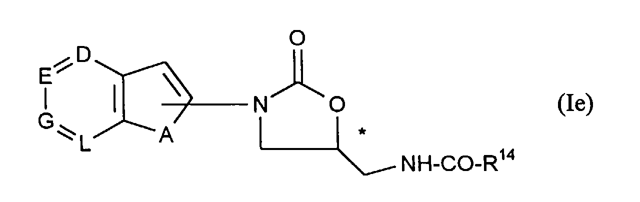 in welcher
A, D, E, G, L und R¹⁴ die oben angegebene Bedeutung haben,
herstellt,
und im Fall R¹ = NR¹²-CS-R^{14'} Verbindungen der allgemeinen Formel (Id) mit Ethyldithiocarboxylaten und Triethylamin und im Fall R¹ = NR¹²-CS-NR¹⁸R¹⁹ mit Thioisocyanaten umsetzt,
und im Fall der S-Oxide eine Oxidation nach üblicher Methode durchführt,
und gegebenenfalls weitere Substituenten oder bereits vorhandene funktionelle Gruppen nach üblichen Methoden, wie beispielsweise Alkylierung, Redoxreaktionen, Substitutionsreaktionen und/oder Verseifungen oder Ein- und Abbau von Schutzgruppen, einführt bzw. derivatisiert,
und gegebenenfalls nach üblichen Methoden die Stereoisomeren trennt.

7. Verbindungen nach einem der Ansprüche 1 bis 5 zur Verwendung bei der Bekämpfung von Krankheiten.

8. Verwendung von Verbindungen nach einem der Ansprüche 1 bis 5 zur Herstellung von Arzneimitteln.

9. Arzneimittel enthaltend Verbindungen nach einem der Ansprüche 1 bis 5.
